(19) Europäisches Patentamt
European Patent Office
Office européen des brevets

(11) **EP 3 493 734 B1**

(12) **EUROPEAN PATENT SPECIFICATION**

(45) Date of publication and mention
of the grant of the patent:
**08.09.2021 Bulletin 2021/36**

(51) Int Cl.:
***A61B 5/021*** *(2006.01)*          *A61B 5/024* *(2006.01)*

(21) Application number: **17836517.7**

(86) International application number:
**PCT/IL2017/050826**

(22) Date of filing: **22.07.2017**

(87) International publication number:
**WO 2018/025257 (08.02.2018 Gazette 2018/06)**

(54) **BLOOD PRESSURE MEASUREMENT USING A WEARABLE DEVICE**

BLUTDRUCKMESSUNG MITHILFE EINER TRAGBAREN VORRICHTUNG

MESURE DE LA PRESSION SANGUINE EN UTILISANT UN DISPOSITIF PORTABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.08.2016 US 201615226881**

(43) Date of publication of application:
**12.06.2019 Bulletin 2019/24**

(73) Proprietor: **ChroniSense Medical Ltd.
Yokneam 2069202 (IL)**

(72) Inventor: **LANGE, Daniel H.
Yokneam 2069202 (IL)**

(74) Representative: **Knöner, Gregor et al
Kahler Käck Mollekopf
Partnerschaft von Patentanwälten mbB
Vorderer Anger 239
86899 Landsberg am Lech (DE)**

(56) References cited:
**US-A1- 2009 163 821     US-A1- 2015 366 469
US-A1- 2017 202 459     US-B1- 6 527 725
US-B2- 8 602 997**

EP 3 493 734 B1

## Description

CROSS-REFERENCE TO RELATED APPLICATIONS

The present application is a Continuation in Part of U.S. Patent Application No. 14/738,666, titled "Monitoring Health Status of People Suffering from Chronic Diseases," filed on June 12, 2015, and is a Continuation in Part of U.S. Patent application No. 14/738,636, titled "Wearable Device Electrocardiogram," filed on June 12, 2015, and is also a Continuation in Part of U.S. Patent Application No. 14/738,711, titled "Pulse Oximetry," filed on June 12, 2015.

FIELD

The present application relates to systems and methods for monitoring the health status of people, and more specifically to systems and methods for continuous or intermittent measurement of non-invasive blood pressure (NIBP).

BACKGROUND

It should not be assumed that any of the approaches described in this section qualify as prior art merely by virtue of their inclusion in this section.
Blood pressure (BP) is one of the basic medical parameters used to diagnose human health condition. The most accurate methods for BP measurements involve insertion of a catheter into a human artery. However, the BP measurements using a catheter are invasive and costly since they require a medical professional to perform the measurements and, typically, can only be performed in a medical facility environment.
Less accurate methods for BP measurements include use of an inflatable cuff to pressurize a blood artery. There are numerous cuff-based portable devices for BP measurements that patients can use at home and do not require assistance of a medical professional. However, cuff-based measurements require inflation and deflation of the inflatable cuff. Therefore, such devices are cumbersome to use and not suitable for ongoing BP measurements.
Some cuff-less devices for BP measurements use an electrical sensor to measure an electrocardiogram (ECG) and optical sensors to measure a photoplethysmogram (PPG). The ECG and PPG can be analyzed to determine pulse transit time (PTT). Because the PTT is in-part inversely proportional to the BP, the BP can in some cases be determined from the PTT using a pre-defined relationship. However, changes in a cardiovascular status of a patient require often re-calibration of PTT based blood pressure measurements. Cuff-less devices can potentially provide continuous monitoring of the BP while imposing a minimal burden on normal activities when worn on various body parts such as a finger, a wrist, or an ankle.

Determining the BP based on the PTT alone may not be sufficiently accurate because of other cardiovascular parameters affecting hemodynamics such as vascular resistance, cardiac output, pulse rate (PR), temperature of a finger (if PPG is measured at the finger), and so forth. To compensate for influences of other parameters, some existing techniques for measuring of BP using the PPG include applying correction factors to account for the vascular resistance and age of patient. The correction factors can be determined by an empirical formula. Some other techniques attempt to determine compensation factors to compensate for various additional influences (for example, contacting force to sensors, nervous activity and cardiac output of patient, and ambient temperature). The compensation factors can be determined using a calibration process. US-2009/163821-A1, US-2015/366469-A1, US-8602997-B2, US-6527725-B1, and US-2017/202459-A1 describe known methods for blood pressure measurements.
However, all currently known methods for cuffless, non-inflatable BP or NIBP monitoring require frequent re-calibration to compensate for unaccounted changes in cardiovascular status of a patient. Therefore, there is a clear need for an NIBP monitoring which can account for changes in the cardiovascular status and does not require a frequent re-calibration.

SUMMARY

This summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.
According to one aspect of the present disclosure, systems and methods for blood pressure measurement are provided. An example method includes simultaneous recording, by a wearable device, of an ECG and a PPG. The PPG is measured at a blood artery. The method includes analyzing, by at least one processor, the ECG and the PPG to determine a PTT, a PR, and a diameter parameter. The diameter parameter includes one of the diameter of the blood artery or a change in the diameter of the blood artery. The method includes determining, by the at least one processor and using a pre-defined model, a BP based on the PTT, PR and diameter parameter. The pre-defined model establishes a relationship between at least the PTT, PR, diameter parameter, and BP.
In some embodiments, the pre-defined model includes regression analysis, wherein the PTT, PR, and diameter parameter are explanatory variables and the BP is a dependent variable.
In certain embodiments, the pre-defined model is trained using statistical data obtained during a calibration process, with the statistical data including the PTT, PR, and diameter parameter measured with the wearable device and corresponding values of the BP measured with an external device.

In some embodiments, the PTT is determined as a shift in time between a certain feature in the ECG and an associated feature in the PPG. The certain feature in the ECG and the associated feature in PPG correspond to the same heartbeat. The feature may include a certain peak or landmark in the ECG or PPG.

In some embodiments, the PTT is determined as a shift in time between a certain waveform of the ECG and an associated waveform in the PPG, with the certain ECG waveform and the associated second PPG waveform corresponding to the same heartbeat.

In some embodiments, the PR is determined as a time period between two peaks associated with two consecutive heart beats in the ECG.

In some embodiments, the PPG includes intensity of a light reflected from the blood artery. In certain embodiments, a wavelength of the light is isosbestic relative to light absorption by blood in the blood artery.

In some embodiments, the change in the diameter of the blood artery is determined using the following equation: $\left(\frac{AC}{DC}\right) = c * (\Delta\ d),$ wherein DC is a direct current component of the PPG, the AC is an alternating current component of the PPG, c is an absorption coefficient of the blood in the blood artery, and $\Delta d$ is the change of the diameter of the blood artery during a heartbeat cycle.

In some embodiments, the blood artery includes a radial artery at a wrist.

According to another example embodiment of the present disclosure, the steps of the method for blood pressure measurement are stored on a non-transitory machine-readable medium comprising instructions, which when implemented by one or more processors perform the recited steps.

Other example embodiments of the disclosure and aspects will become apparent from the following description taken in conjunction with the following drawings.

BRIEF DESCRIPTION OF THE DRAWINGS

Embodiments are illustrated by way of example and not limitation in the figures of the accompanying drawings, in which like references indicate similar elements.

FIG. 1 is a block diagram showing an example system for performing a blood pressure measurement using a wearable device.

FIG. 2 is a block diagram showing components of an example device for performing blood pressure measurement.

FIG. 3 is block diagram illustrating an example device for measuring arterial blood pressure from a wrist.

FIG. 4 shows an example plot of an ECG and an example plot of a PPG.

FIG. 5 shows an example plot of a PPG and an example plot of a blood vessel diameter.

FIG. 6 is a flow chart showing an example method for performing blood pressure measurement.

FIG. 7 shows a diagrammatic representation of a computing device for a machine, within which a set of instructions for causing the machine to perform any one or more of the methodologies discussed herein can be executed.

DETAILED DESCRIPTION

The following detailed description includes references to the accompanying drawings, which form a part of the detailed description. The drawings show illustrations in accordance with exemplary embodiments. These exemplary embodiments, which are also referred to herein as "examples," are described in enough detail to enable those skilled in the art to practice the present subject matter. The embodiments can be combined, other embodiments can be utilized, or structural, logical and electrical changes can be made without departing from the scope of what is claimed. The following detailed description is, therefore, not to be taken in a limiting sense, and the scope is defined by the appended claims and their equivalents.

The present disclosure provides systems and methods for performing blood pressure measurement. Embodiments of the present disclosure allow for continuous or intermittent measuring of blood pressure of a patient in a non-intrusive manner while, for example, the patient is at home, at work, outdoors, traveling, or is located at some other stationary or mobile environment. Embodiments of the present disclosure include a wearable device. The wearable device can be worn at a wrist, ankle, chest, neck, or positioned at other sites on a human body. The wearable device can allow measuring blood pressure of the patient without requiring the patient to take an active role in the process. The blood pressure data collected over an extended period of time can be analyzed to detect and track trends in medical parameters and to make conclusions concerning symptoms and a progression of one or more chronic diseases from which the patient may suffer.

According to some example embodiments, methods for performing blood pressure measurements include recording simultaneously, by a wearable device, an ECG and a PPG. The PPG is measured at a blood artery. The method further includes analyzing, by at least one processor, ECG and PPG to determine a PTT, a PR, and a diameter parameter. The diameter parameter includes one of the diameter of the blood artery or a change in the diameter of the blood artery. The method further includes determining, by the at least one processor and using a pre-defined model, including at least a BP based on PTT, PR and the diameter parameter. The pre-defined model establishes a relationship between the at least a PTT, PR, the diameter parameter, and BP.

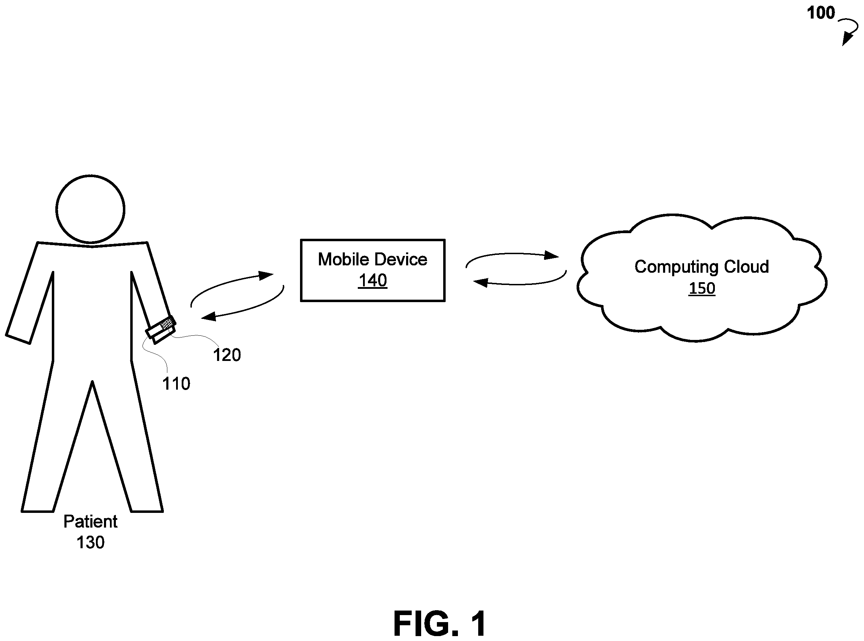

Referring now to FIG. 1, an example system 100 for performing blood pressure measurements is shown. The system 100 can include at least a wearable device 110. The wearable device 110 can include sensors 120. In some embodiments, the wearable device 110 is worn by a patient 130 (for example, on a wrist, ankle, earlobe, neck, chest, fingertip, and the like) for an extended period of time. In various embodiments, the wearable device 110 can be carried out as a watch, a bracelet, a wristband, a belt, a neck band, and the like.

The wearable device 110 can be operable to constantly collect, via sensors 120, sensor data from a patient 130. Based on the sensor data, the wearable device 110 can be operable to provide PPG and ECG. The PPG and ECG can be further used to obtain further medical parameters (for example, pulse rate, pulse transition time, blood pressure, and so forth).

In some embodiments, the system 100 includes a mobile device 140. The mobile device 140 can be communicatively coupled to the wearable device 110. In various embodiments, the mobile device 140 is operable to communicate with the wearable device 110 via a wireless connection such as, for example, Wi-Fi, Bluetooth, Infrared (IR), and the like. The mobile device 140 can include a mobile phone, a smart phone, a phablet, a tablet computer, a notebook, and so forth. The mobile device 140 can be operable to receive the sensor data and analyze the sensor data to provide ECG and PPG.

In further embodiments, the system 100 may include a cloud-based computing resource also referred to as a computing cloud 150. In some embodiments, the computing cloud 150 includes one or more server farms/clusters comprising a collection of computer servers and is co-located with network switches and/or routers. In certain embodiments, the mobile device 140 is communicatively coupled to the computing cloud 150. The mobile device 140 can be operable to send the sensor data to the computing cloud 150 for further analysis (for example, for extracting medical parameters from the ECG and PPG and storing the results). The computing cloud 150 can be operable to run one or more applications and to provide reports regarding a health status of the patient, based on trends in medical parameters over time.

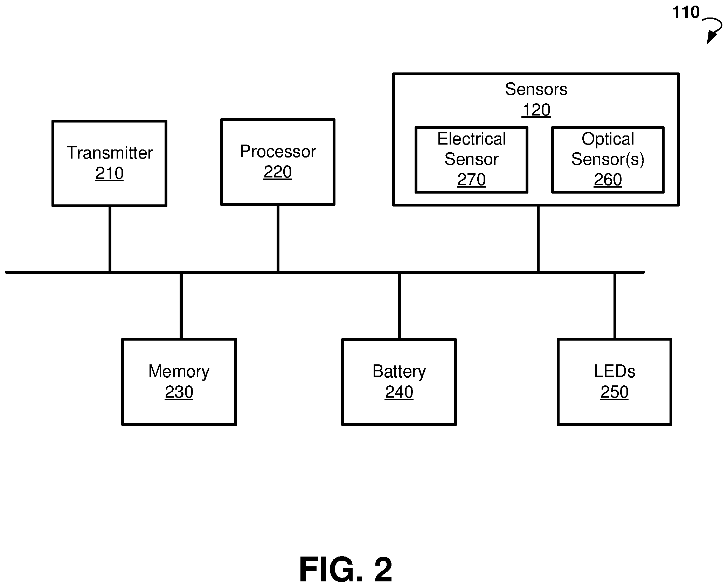

FIG. 2 is a block diagram illustrating components of wearable device 110, according to an example embodiment. The example wearable device 110 includes a transmitter 210, a processor 220, memory 230, a battery 240, light-emitting diodes (LEDs) 250, optical sensor(s) 260, and electrical sensor 270. The wearable device 110 may comprise additional or different components to provide a particular operation or functionality. Similarly, in other embodiments, the wearable device 110 includes fewer components that perform similar or equivalent functions to those depicted in FIG. 2.

The transmitter 210 can be configured to communicate with a network such as the Internet, a Wide Area Network (WAN), a Local Area Network (LAN), a cellular network, and so forth, to send data streams (for example sensor data, PPG data, and messages).

The processor 220 can include hardware and/or software, which is operable to execute computer programs stored in memory 230. The processor 220 can use floating point operations, complex operations, and other operations, including processing and analyzing data obtained from electrical sensor 270 and optical sensor(s) 260.

In some embodiments, the battery 240 is operable to provide electrical power for operation of other components of the wearable device 110. In some embodiments, the battery 240 is a rechargeable battery. In certain embodiments, the battery 240 is recharged using an inductive charging technology.

In various embodiments, the LEDs 250 are operable to emit light signals. The light signals can be of a red wavelength (typically 660 nm) or infrared wavelength (660 nm). Each of the LEDs 250 is activated separately and accompanied by a "dark" period where neither of the LEDs 250 is on to obtain ambient light levels. In some embodiments, a single LED 250 can be used to emit both the infrared and red light signals. The lights can be absorbed by human blood (mostly by hemoglobin). The oxygenated hemoglobin absorbs more infrared light while deoxygenated hemoglobin absorbs more red light. Oxygenated hemoglobin allows more red light to pass through while deoxygenated hemoglobin allows more infrared light to pass through. In some embodiments of the present disclosure, the LEDs 250 are also operable to emit light signals of isosbestic wavelengths (typically 810nm and 520nm). Both oxygenated hemoglobin and deoxygenated hemoglobin absorb the light of the isosbestic wavelengths equally.

The optical sensor(s) 260 (typically a photodiode) can receive light signals modulated by human tissue. Intensity of the modulated light signal represents a PPG. Based on the changes in the intensities of the modulated light signals, one or more medical parameters, such as, for example, oxygen saturation, arterial blood flow, pulse rate, and respiration, can be determined.

The LEDs 250 and optical sensor(s) 260 can be utilized in either a transmission or a reflectance mode for pulse oximetry. In the transmission mode, the LEDs 250 and optical sensor(s) 260 are typically attached or clipped to a translucent body part (e.g., a finger, toe, and earlobe). The LEDs 250 are located on one side of the body part while the optical sensor(s) 260 are located directly on the opposite site. The light passes through the entirety of the body part, from one side to the other, and is thus modulated by the pulsating arterial blood flow. In the reflectance mode, the LEDs 250 and optical sensor(s) 260 are located on the same side of the body part (e.g. a forehead, a finger, and a wrist), and the light is reflected from the skin and underlying near-surface tissues back to the optical sensor(s) 260.

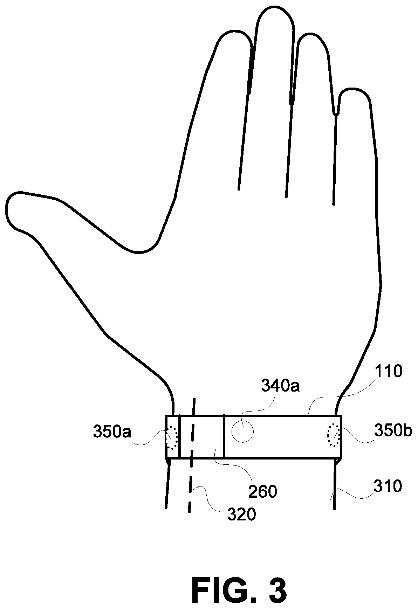

FIG. 3 is a block diagram illustrating an example wearable device 110 placed around a wrist of a patient. In the example of FIG. 3, the wearable device 110 is carried out in a shape of a watch, a ring, and/or a bracelet.

The electrical sensor 270 can include a differential amplifier operable to measure the electrical signal from the wrist. The electrical sensor 270 can include two active amplifier input plates embedded in the wearable device at opposite ends. In some embodiments, the first input plate (not shown) can be placed above the outer side of the wrist, and the second input plate 340a can be placed beneath the inner side of the wrist. Alternatively or additionally, in other embodiments, input plates 350a and 350b can be placed in contact with, respectively, the left and right sides of the wrist.

In some embodiments, the optical sensor(s) 260 can be placed beneath a pulsating artery travelling along the arm and into a wrist 310. In some embodiments, a radial artery 320 passing in the inner wrist is used for measurements by the optical sensor(s) 260. In other embodiments, other arteries such as the ulnar artery, may be used. An external light source generating constant lighting can be used to radiate the pulsating artery. A beam reflected from the pulsating artery can be intercepted by the optical sensor(s) 260. In certain embodiments, a light of isosbestic wavelength is used to radiate the pulsating artery.

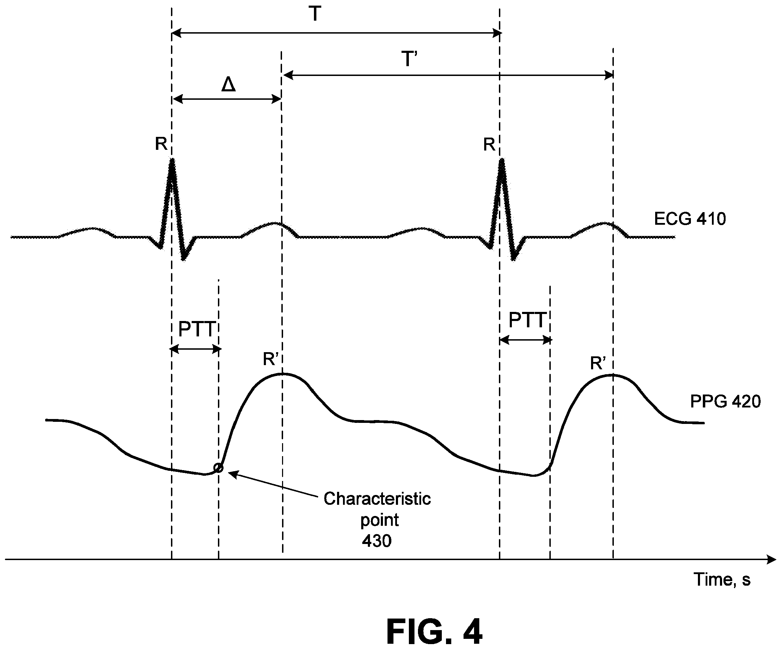

FIG. 4 shows plots of an example an example plot of an ECG 410, and an example plot of a PPG 420. The ECG 410 can be recorded with electrical sensor 270 using input plates placed on the wearable device 110. The ECG 410 can include R peaks corresponding to heart beats. Taking measurements from a single hand or a single wrist is challenging because the difference in voltages between measured locations is miniscule. The electrical signal measured at the wrist can include an ECG 410 and a noise. The noise can be caused by muscle activity, patient movements, and so forth. The noise component can be larger than the ECG. In some embodiments, the signal-to-noise ratio (SNR) is in the range of -40dB to -60dB. An example method for measuring a "clean" ECG from a wrist is described in U.S. Patent application No. 14/738,666, titled "Wearable Device Electrocardiogram," filed on June 12, 2015.

The PPG 420 can be obtained by sensing a change in the color of skin. The change of the skin color is caused by a blood flow in a pulsating artery. In some embodiments, the PPG 420 can include peaks R' related to the heart beats. Since it takes a time for blood to flow from the heart to the wrist, the peaks R' are shifted by time periods Δ relative to the heart beats R in ECG 420. In some embodiments, shifts Δ can be measured as shift of a waveform of PPG (complex of PPG corresponding to period T' in FIG. 4) relative to a waveform of ECG (complex of ECG corresponding to period T in FIG. 4).

In various embodiments, ECG 410 and PPG 420 are used to estimate a PTT. In some embodiments, PTT is defined as a time interval between the R peak in ECG 410 and characteristic point 430 located at the bottom of the PPG 420. PTT is a parameter which inversely correlates to BP. PTT decreases as BP increases and PTT increases as BP decreases. Therefore, PTT can be used to estimate BP. In some embodiments, a regression equation can be derived to establish a relation between PTT and BP. The regression equation can be established for both systolic BP and diastolic BP. Alternatively in other embodiments, other mathematical models, such as neural networks, may be used to establish the relation between the PTT and BP.

The location of characteristic point 430 can be uncertain or hard to detect. For example, a shape of PPG at a foot can be diffused when a pulse rate is high. Therefore, in some embodiments, when location of characteristic point 430 is uncertain or hard to detect, shifts between specific features of the ECG and PPG (such as certain landmarks or peaks) corresponding to the same heartbeat are used as an estimate for PTT. In certain embodiments, PTT is estimated based on shifts between waveforms of ECG and PPG corresponding to the same heartbeat.

PTT depends on the shape and cross-section area of a blood vessel (for example, a pulsating artery at which measurement is performed) since speed of blood travelling through the blood vessel depends on the cross-section area of the blood vessel and blood pressure.

According to various embodiments of the present disclosure, ECG and PPG are used to estimate PTT, PR, and diameter of the blood vessel or a change in the diameter of the blood vessel. In some embodiments, PTT, PR, and the diameter of the blood vessel or the change in the diameter of the blood vessel are then used to estimate BP. In some embodiments, PTT is determined based on ECG and PPG. PR can be found using a time period between two consecutive peaks in ECG or two consecutive peaks in PPG. In some embodiments, the diameter of the blood vessel or the change in the diameter of the blood vessel can be estimated using PPG.

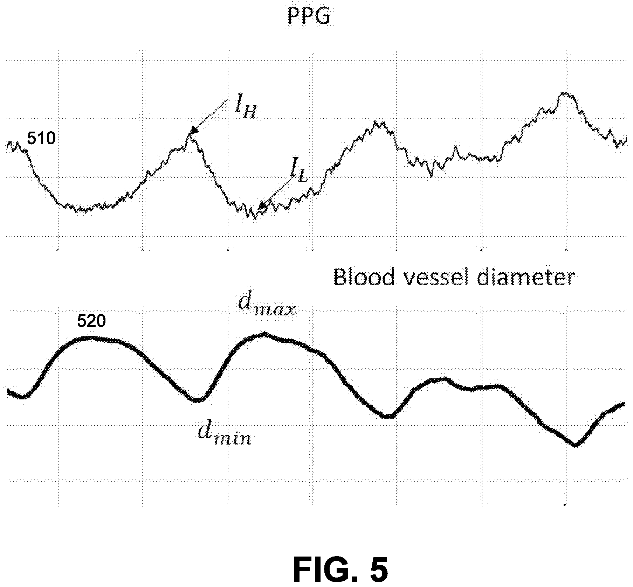

FIG. 5 shows an example plot of PPG 510 and an example plot of blood vessel diameter 520. The PPG 510 represents the intensity I of the light signal as modulated by a human tissue mostly due to a blood flow in the blood vessel. The high peaks (maximums) $I_H$ of PPG 510 correspond to the low peaks $d_{min}$ of the blood vessel diameter 520, and the low peaks $I_L$ of the PPG 510 correspond to the high peaks $d_{max}$ of the blood vessel diameter 520.

In some embodiments, the detected PPG signal I, which is the intensity of light signal reflected from pulsating tissue, is modeled as follows:

$$I(t) = I_0 * F * e^{-c*d(t)} \ (1).$$

In formula (1), $I_0$ represents an incident light intensity, F indicates the absorption by pulsatile tissue, $d(t)$ represents (arterial) blood vessel diameter, and c is overall absorption coefficient of blood hemoglobin derived from a mixture of both oxygen-saturated and non-oxygen saturated hemoglobin. Each of oxygen-saturated and non-oxygen saturated hemoglobin has its own particular value of absorption coefficient c for a particular wavelength of emitted light. Therefore, according to some embodiments, a light of isosbestic wavelength is used to radiate the pulsatile tissue allowing absorption coefficient c remain constant and independent of SpO2 oxygen saturation. The light absorption at the isosbestic wavelength is independent of SpO2 oxygen saturation because when a light of an isosbestic wavelength is used, the reflection from the oxygenized blood is the same as reflection from the non-oxygenized blood. In some embodiments, the isosbestic wavelength includes a near infrared wavelength *810nm* (NIR) and a green wavelength *520nm (green)*. The NIR wavelength is more suitable for deeper vessels as it has deeper penetration while the green wavelength is more suitable for shallow vessels.

As shown in FIG. 5, the blood vessel diameter 520 changes periodically with the rhythm of the heart rate. The low peaks of the blood vessel diameter $d_{min}$ correspond to the minimums of the absorption of the light by the blood and the high peaks of the light intensity $I_H$. The high peaks of the blood vessel diameter $d_{max}$ correspond to maximum absorption of the light by blood and the lowest peaks of the light intensity $I_L$. In some embodiments, the low peaks of the blood vessel diameter $d_{min}$ can be considered to be constant as they reflect lowest diastole. The high peaks of the blood vessel diameter $d_{max}$ may vary relatively slowly due to, for example, fluctuations of blood pressure.

In some embodiments, it can be assumed that

$$I(t) \approx I_0 * F * (1 - c * d(t)) \ (2).$$

Denoting further direct current (DC) component of PPG

$$DC = I_0 * F \ (3)$$

and alternating current (AC) component

$$AC = I_0 * F * c * d(t) \ (4),$$

an equation for determining blood vessel diameter d(t) can be written as:

$$\left(\frac{AC}{DC}\right) = c * d(t) \ (5).$$

In equation (5), the AC component and DC component are found from PPG and absorption coefficient c is known. In some embodiments, change $d(t)_{max}-d(t)_{min}$ is used to estimate BP.

In other embodiments, BP is calculated from measured PTT, PR, and the diameter of the blood vessel or a change thereof using a pre-defined model. The pre-defined model describes a relationship between PTT, PR, and the diameter of the blood vessel and BP. In some embodiments, the pre-defined model is determined using statistical data collected during a calibration process. During the calibration process, a patient can wear the wearable device 110 to measure PTT, PR, and the diameter of the blood vessel or a change in the diameter of the blood vessel. Simultaneously, BP can be measured using an external device (for example, a conventional device for BP measurement). The calibration can be performed once at first usage of the wearable device 110 by a particular patient, and requires at least a single simultaneous measurement by the wearable device 110 and the external device. In other embodiments, several simultaneous measurements should be made to calibrate the wearable device 110 in a range of blood pressure values. The range of blood pressure values can be achieved by taking measurements at either or all the following: different times (hours of a day), different physical states of a patient, and different emotional states of the patient. Alternatively, lowering or elevating the arm and taking local blood pressure at the wrist with both an external device and the wearable device 110 can provide an effective means for mapping the PTT, PR, and diameter of the blood vessel or a change in the diameter of the blood vessel to a wide range of blood pressure values.

In some embodiments, the pre-defined model includes a three-dimensional model, wherein PTT, PR and the diameter of the blood vessel or a change in the diameter of the blood vessel are explanatory variables and systolic blood pressure is a dependent variable. Similarly, another three-dimensional model can be used to establish mathematical relationships between PTT, PR and diameter of blood vessel or a change in the diameter of the blood vessel as explanatory variables and diastolic blood pressure as a dependent variable.

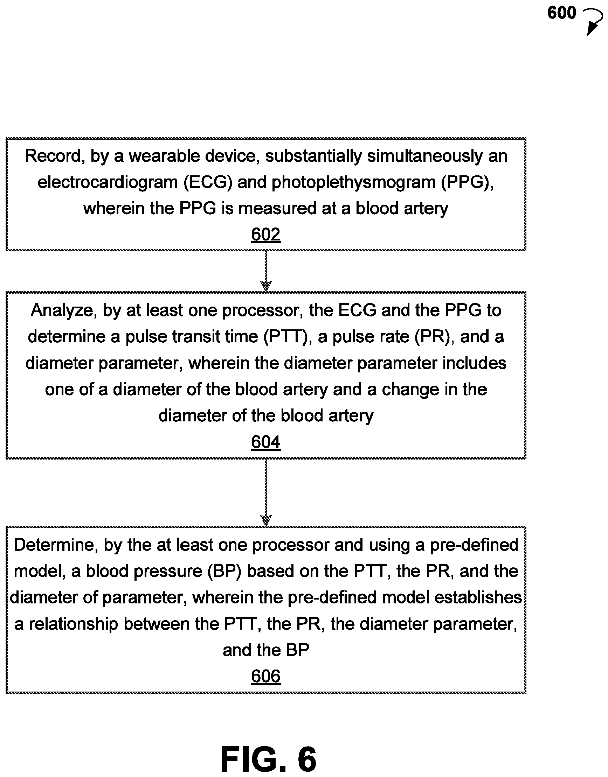

FIG. 6 is a flow chart showing steps of a method 600 for performing BP measurement, according to some embodiments. The method 600 can be implemented using wearable device 110 described in FIG. 2 and 3 and system 100 described in FIG. 1. The method 600 may commence in block 602 with substantially simultaneous recording, by a wearable device, an ECG and a PPG. In some embodiments, PPG is measured at a blood artery. In some embodiments, ECG and PPG are recorded at a wrist.

In block 604, the method 600 proceeds with analyzing ECG and PPG to determine a PTT, a PR, and a diameter parameter. The diameter parameter may include a diameter of the blood artery or a change in the diameter of the blood artery. In block 606, the method 600 determines, based on PTT, PR, and the diameter parameter, BP using a pre-defined model. The pre-defined model establishes a relationship between the PTT, the PR, the diameter parameter, and the BP. In some embodiments, analysis of ECG and PPG and determination of PTT, the PR, the diameter parameter, and BP is performed locally using processor of the wearable device. In other embodiments, analysis of ECG and PPG and determination of PTT, the PR, the diameter parameter, and BP can be carried out remotely by a mobile device connected to the wearable device or in a computing cloud.

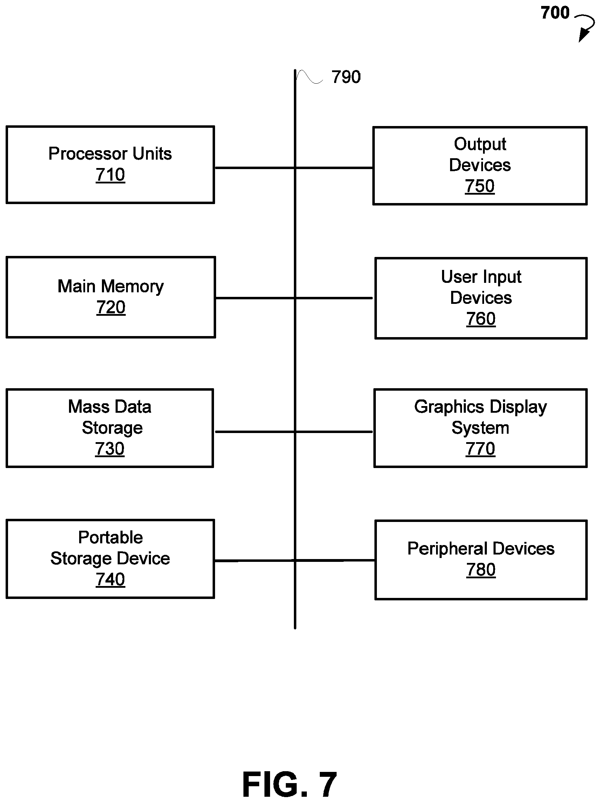

FIG. 7 illustrates a computer system 700 that may be used to implement embodiments of the present disclosure, according to an example embodiment. The computer system 700 may serve as a computing device for a machine, within which a set of instructions for causing the machine to perform any one or more of the methodologies discussed herein can be executed. The computer system 700 can be implemented in the contexts of the likes of computing systems, networks, servers, or combinations thereof. The computer system 700 includes one or more processor units 710 and main memory 720. Main memory 720 stores, in part, instructions and data for execution by processor units 710. Main memory 720 stores the executable code when in operation. The computer system 700 further includes a mass data storage 730, a portable storage device 740, output devices 750, user input devices 760, a graphics display system 770, and peripheral devices 780. The methods may be implemented in software that is cloud-based.

The components shown in FIG. 7 are depicted as being connected via a single bus 790. The components may be connected through one or more data transport means. Processor units 710 and main memory 720 are connected via a local microprocessor bus, and mass data storage 730, peripheral devices 780, the portable storage device 740, and graphics display system 770 are connected via one or more I/O buses.

Mass data storage 730, which can be implemented with a magnetic disk drive, solid state drive, or an optical disk drive, is a non-volatile storage device for storing data and instructions for use by processor units 710. Mass data storage 730 stores the system software for implementing embodiments of the present disclosure for purposes of loading that software into main memory 720.

The portable storage device 740 operates in conjunction with a portable non-volatile storage medium, such as a floppy disk, compact disk (CD), Digital Versatile Disc (DVD), or USB storage device, to input and output data and code to and from the computer system 700. The system software for implementing embodiments of the present disclosure is stored on such a portable medium and input to the computer system 700 via the portable storage device 740.

User input devices 760 provide a portion of a user interface. User input devices 760 include one or more microphones, an alphanumeric keypad, such as a keyboard, for inputting alphanumeric and other information, or a pointing device, such as a mouse, a trackball, stylus, or cursor direction keys. User input devices 760 can also include a touchscreen. Additionally, the computer system 700 includes output devices 750. Suitable output devices include speakers, printers, network interfaces, and monitors.

Graphics display system 770 includes a liquid crystal display or other suitable display device. Graphics display system 770 receives textual and graphical information and processes the information for output to the display device. Peripheral devices 780 may include any type of computer support device to add additional functionality to the computer system.

The components provided in the computer system 700 of FIG. 7 are those typically found in computer systems that may be suitable for use with embodiments of the present disclosure and are intended to represent a broad category of such computer components that are well known in the art. Thus, the computer system 700 can be a personal computer, handheld computing system, telephone, mobile computing system, workstation, tablet, phablet, mobile phone, server, minicomputer, mainframe computer, or any other computing system. The computer may also include different bus configurations, networked platforms, multi-processor platforms, and the like. Various operating systems may be used including UNIX, LINUX, WINDOWS, MAC OS, PALM OS, ANDROID, IOS, QNX, TIZEN and other suitable operating systems.

It is noteworthy that any hardware platform suitable for performing the processing described herein is suitable for use with the embodiments provided herein. Computer-readable storage media refer to any medium or media that participate in providing instructions to a central processing unit, a processor, a microcontroller, or the like. Such media may take forms including, but not limited to, non-volatile and volatile media such as optical or magnetic disks and dynamic memory, respectively. Common forms of computer-readable storage media include a floppy disk, a flexible disk, a hard disk, magnetic tape, any other magnetic storage medium, a CD Read Only Memory disk, DVD, Blu-ray disc, any other optical storage medium, RAM, Programmable Read-Only Memory, Erasable Programmable Read-Only Memory, Electronically Erasable Programmable Read-Only Memory, flash memory, and/or any other memory chip, module, or cartridge.

In some embodiments, the computer system 700 may be implemented as a cloud-based computing environment, such as a virtual machine operating within a computing cloud. In other embodiments, the computer system 700 may itself include a cloud-based computing environment, where the functionalities of the computer system 700 are executed in a distributed fashion. Thus, the computer system 700, when configured as a computing cloud, may include pluralities of computing devices in various forms, as will be described in greater detail below.

In general, a cloud-based computing environment is a resource that typically combines the computational power of a large grouping of processors (such as within web servers) and/or that combines the storage capacity of a large grouping of computer memories or storage devices. Systems that provide cloud-based resources may be utilized exclusively by their owners or such systems may be accessible to outside users who deploy applications within the computing infrastructure to obtain the benefit of large computational or storage resources.

The cloud may be formed, for example, by a network of web servers that comprise a plurality of computing devices, such as the computer system 700, with each server (or at least a plurality thereof) providing processor and/or storage resources. These servers may manage workloads provided by multiple users (e.g., cloud resource customers or other users). Typically, each user places workload demands upon the cloud that vary in real-time, sometimes dramatically. The nature and extent of these variations typically depends on the type of business associated with the user.

Thus, methods and systems for performing pulse oximetry have been described. Although embodiments have been described with reference to specific example embodiments, it will be evident that various modifications and changes can be made to these example embodiments without departing from the scope of the present application. Accordingly, the specification and drawings are to be regarded in an illustrative rather than a restrictive sense.

**Claims**

1.  A method for performing a blood pressure, BP, measurement, the method comprising:

    recording substantially simultaneously, by a wearable device (110), an electrocardiogram, ECG, and photoplethysmogram, PPG, wherein the PPG is measured at a blood artery;
    analyzing, by at least one processor (220, 710), the ECG and the PPG to determine a pulse transit time, PTT, a pulse rate, PR, and a diameter parameter, wherein the diameter parameter includes one of a diameter of the blood artery and a change in the diameter of the blood artery; and
    determining, by the at least one processor (220, 710) and using a pre-defined model, a BP based on the PTT, the PR, and the diameter parameter, wherein the pre-defined model establishes a relationship between the PTT, the PR, the diameter parameter, and the BP.

2.  The method of claim 1, wherein the pre-defined model includes a regression analysis, wherein the PTT, the PR, and the diameter parameter are explanatory variables and the BP is a dependent variable.

3.  The method of claim 1 or 2, wherein the pre-defined model is trained using statistical data obtained in a calibration process, the statistical data including the PTT, the PR, and the diameter parameter measured with the wearable device (110) and corresponding values of the BP measured with an external device.

4.  The method of any one of the preceding claims, wherein the PTT is determined as a shift in time between a certain feature (R) in the ECG and an associated feature (430) in the PPG, the certain feature (R) and the associated feature (430) corresponding to a same heartbeat.

5.  The method of any one of claims 1-3, wherein the PTT is determined as a shift in time between a first waveform of the ECG and a second waveform in the PPG, the first waveform and the second waveform corresponding to a same heartbeat.

6.  The method of any one of the preceding claims, wherein the PR is determined as a time period between two consecutive peaks in ECG.

7.  The method of any one of the preceding claims, wherein the PPG includes an intensity of a light reflected from the blood artery, wherein a wavelength of the light is preferably isosbestic relative to a light absorption by blood in the blood artery.

8.  The method of any one of the preceding claims, wherein the change in the diameter of the blood artery is determined using equation $\left(\frac{AC}{DC}\right) = c * \Delta\, d,$ wherein DC is a direct current component of the PPG, AC is an alternative current component of the PPG, c is an absorption coefficient of blood in the blood artery, and $\Delta\, d$ is the change of the diameter of the blood artery.

9.  The method of any one of the preceding claims, wherein the blood artery is a radial artery (320) at a wrist (310).

10. A system for performing a pulse oximetry, the system comprising:

    a wearable device (110) operable to record simultaneously an electrocardiogram, ECG, and photoplethysmogram, PPG, wherein the PPG is measured at a blood artery; and
    at least one processor (220, 710) communicatively coupled to the wearable device (110) and operable to:

        analyze the ECG and the PPG to determine a pulse transit time, PTT, a pulse rate, PR, and a diameter parameter, the diameter parameter including one of diameter of the blood artery and a change in the diameter of the blood artery; and

determine, based on the PTT, the PR, and the diameter parameter, a blood pressure, BP, using a pre-defined model, wherein the pre-defined model establishes a relationship between the PTT, the PR, the diameter parameter, and the BP.

11. The system of claim 10, wherein the at least one processor (220, 710) is operable to perform the method of any one of claims 2-6.

12. The system of claim 10 or 11, wherein the PPG includes intensity of a light reflected from the blood artery.

13. The system of claim 12, wherein a wavelength of the light is isosbestic relative to light absorption by blood in the blood artery.

14. The system of any one of claims 10-13, wherein the change in the diameter of the blood artery is determined using equation $\left(\frac{AC}{DC}\right) = c * \Delta\, d,$ wherein DC is a direct current component of the PPG, AC is an alternative current component of the PPG, *c* is an absorption coefficient of blood in the blood artery, and $\Delta$ d is the change of the diameter of the blood artery.

15. A non-transitory computer-readable storage medium having embodied thereon instructions, which when executed by at least one processor (220, 710), perform steps of a method according to any one of claims 1-9.

**Patentansprüche**

1. Verfahren für das Ausführen einer Blutdruck-, BP-, Messung, wobei das Verfahren umfasst:

Aufzeichnen, im Wesentlichen gleichzeitig, durch eine tragbare Vorrichtung (110), eines Elektrokardiogramms, ECG, und eines Photoplethysmogramms, PPG, wobei das PPG an einer Blutarterie gemessen wird;
Analysieren, durch mindestens einen Prozessor (220, 710), des ECG und des PPG, um eine Pulslaufzeit, PTT, eine Pulsrate, PR, und einen Durchmesserparameter festzustellen, wobei der Durchmesserparameter eines von einem Durchmesser der Blutarterie und einer Änderung des Durchmessers der Blutarterie beinhaltet; und
Bestimmen, durch den mindestens einen Prozessor (220, 710) und unter Verwendung eines vordefinierten Modells, eines BP basierend auf der PTT, der PR, und dem Durchmesserparameter, wobei das vordefinierte Modell eine Beziehung zwischen der PTT, der PR, dem Durchmesserparameter, und dem BP herstellt.

2. Verfahren nach Anspruch 1, wobei das vordefinierte Modell, eine Regressionsanalyse beinhaltet, wobei die PTT, die PR, und der Durchmesserparameter erklärende Variablen sind und der BP eine abhängige Variable ist.

3. Verfahren nach Anspruch 1 oder 2, wobei das vordefinierte Modell unter Verwendung statistischer Daten, die in einem Kalibrationsverfahren gewonnen werden, trainiert wird, wobei die statistischen Daten die PTT, die PR, und den Durchmesserparameter, gemessen mit der tragbaren Vorrichtung, und die zugehörigen Werte des BP, gemessen mit einer externen Vorrichtung, beinhalten.

4. Verfahren nach einem der vorstehenden Ansprüche, wobei die PTT als eine zeitliche Verschiebung zwischen einem bestimmten Merkmal (R) in dem ECG und einem in Verbindung stehenden Merkmal (430) in dem PPG bestimmt wird, wobei das bestimmte Merkmal (R) und das in Verbindung stehende Merkmal (430) dem gleichen Herzschlag zugeordnet sind.

5. Verfahren nach einem der Ansprüche 1-3, wobei die PTT als eine zeitliche Verschiebung zwischen einer ersten Wellenform des ECG und einer zweiten Wellenform in dem PPG bestimmt wird, wobei die erste Wellenform und die zweite Wellenform dem gleichen Herzschlag zugeordnet sind.

6. Verfahren nach einem der vorstehenden Ansprüche, wobei die PR als eine Zeitspanne zwischen zwei aufeinanderfolgenden Spitzen im ECG bestimmt wird.

7. Verfahren nach einem der vorstehenden Ansprüche, wobei das PPG eine Intensität eines Lichts, das von der Blutarterie reflektiert wird, beinhaltet, wobei eine Wellenlänge des Lichts bevorzugt isobestisch relativ zur Lichtabsorption durch Blut in der Blutarterie ist.

8. Verfahren nach einer der vorstehenden Ansprüche, wobei die Änderung des Durchmessers der Blutarterie unter Verwendung der Gleichung $\left(\frac{AC}{DC}\right) = c * \Delta\, d$ bestimmt wird, wobei *DC* eine Gleichstromkomponente des PPG, *AC* eine Wechselstromkomponente des PPG, c ein Absorptionskoeffizient des Blutes in der Blutarterie, und $\Delta$ d die Änderung des Durchmessers der Blutarterie ist.

9. Verfahren nach einem der vorstehenden Ansprüche, wobei die Blutarterie eine Radialarterie (320) an einem Handgelenk (310) ist.

10. System für das Ausführen einer Pulsoxymetrie, wobei das System umfasst:

eine tragbare Vorrichtung (110), die betreibbar ist, um gleichzeitig ein Elektrokardiogramm, ECG, und ein Photoplethysmogramm, PPG, aufzuzeichnen, wobei das PPG an einer Blutarterie gemessen wird; und
mindestens einen Prozessor (220, 710), der kommunikativ mit der tragbaren Vorrichtung (110) gekoppelt ist und betreibbar ist, um:

das ECG und das PPG zu analysieren, um eine Pulslaufzeit, PTT, eine Pulsrate, PR, und einen Durchmesserparameter zu bestimmen, wobei der Durchmesserparameter eines von einem Durchmesser der Blutarterie und einer Änderung des Durchmessers der Blutarterie beinhaltet; und, basierend auf der PTT, der PR, und dem Durchmesserparameter, einen Blutdruck, BP, unter Verwendung eines vordefinierten Modells zu bestimmen, wobei das vordefinierte Modell eine Beziehung zwischen der PTT, der PR, dem Durchmesserparameter, und dem BP herstellt.

11. System nach Anspruch 10, wobei der mindestens eine Prozessor (220, 710) fähig ist die Methode nach einem der Ansprüche 2-6 auszuführen.

12. System nach Anspruch 10 oder 11, wobei das PPG Intensität eines Lichts, das von der Blutarterie reflektiert wird, beinhaltet.

13. System nach Anspruch 12, wobei eine Wellenlänge des Lichts isobestisch relativ zur Lichtabsorption durch Blut in der Blutarterie ist.

14. System nach einem der Ansprüche 10-13, wobei die Änderung des Durchmessers der Blutarterie unter Verwendung der Gleichung $\left(\frac{AC}{DC}\right) = c * \Delta\, d$ bestimmt wird, wobei *DC* eine Gleichstromkomponente des PPG, *AC* eine Wechselstromkomponente des PPG, c ein Absorptionskoeffizient des Blutes in der Blutarterie, und $\Delta$ d die Änderung des Durchmessers der Blutarterie ist.

15. Nichtflüchtiges computerlesbares Speichermedium, das darauf Anweisungen enthält, die, sobald sie von mindestens einem Prozessor (220, 710) ausgeführt werden, Schritte eines Verfahrens entsprechend einem der Ansprüche 1-9 ausführen.

**Revendications**

1. Procédé pour effectuer une mesure de la pression sanguine, BP, le procédé comprenant :

enregistrer de manière essentiellement simultanée, par un dispositif portable (110), un électrocardiogramme, ECG, et un photopléthysmogramme, PPG, dans lequel le PPG est mesuré au niveau d'une artère sanguine ;
analyser, par au moins un processeur (220, 710), l'ECG et le PPG pour déterminer un temps de transit du pouls, PTT, une fréquence du pouls, PR, et un paramètre de diamètre, dans lequel le paramètre de diamètre comporte l'un d'un diamètre de l'artère sanguine et d'un changement du diamètre de l'artère sanguine ; et
déterminer, par l'au moins un processeur (220, 710) et en utilisant un modèle prédéfini, une BP sur la base du PTT, de la PR et du paramètre de diamètre, dans lequel le modèle prédéfini établit une relation entre le PTT, la PR, le paramètre de diamètre et la BP.

2. Procédé de la revendication 1, dans lequel le modèle prédéfini comporte une analyse de régression, dans lequel le PTT, la PR et le paramètre de diamètre sont des variables explicatives et la BP est une variable dépendante.

3. Procédé de la revendication 1 ou 2, dans lequel le modèle prédéfini est appris en utilisant des données statistiques obtenues dans un processus d'étalonnage, les données statistiques comportant le PTT, la PR et le paramètre de diamètre mesurés avec le dispositif portable (110) et des valeurs correspondantes de la BP mesurée avec un dispositif externe.

4. Procédé de l'une quelconque des revendications précédentes, dans lequel le PTT est déterminé comme un décalage dans le temps entre une certaine caractéristique (R) dans l'ECG et une caractéristique associée (430) dans le PPG, la certaine caractéristique (R) et la caractéristique associée (430) correspondant à un même battement du cœur.

5. Procédé de l'une quelconque des revendications 1 à 3, dans lequel le PTT est déterminé comme un décalage dans le temps entre une première forme d'onde de l'ECG et une deuxième forme d'onde dans le PPG, la première forme d'onde et la deuxième forme d'onde correspondant à un même battement du cœur.

**6.** Procédé de l'une quelconque des revendications précédentes, dans lequel la PR est déterminée comme une période de temps entre deux pics consécutifs dans l'ECG.

**7.** Procédé de l'une quelconque des revendications précédentes, dans lequel le PPG comporte une intensité d'une lumière réfléchie par l'artère sanguine, dans lequel une longueur d'onde de la lumière est de préférence isosbestique par rapport à une absorption de lumière par le sang dans l'artère sanguine.

**8.** Procédé de l'une quelconque des revendications précédentes, dans lequel le changement du diamètre de l'artère sanguine est déterminé en utilisant l'équation $\left(\frac{AC}{DC}\right) = c * \Delta\,d$ , dans laquelle DC est une composante de courant continu du PPG, AC est une composante de courant alternatif du PPG, c est un coefficient d'absorption du sang dans l'artère sanguine, et $\Delta$ d est le changement du diamètre de l'artère sanguine.

**9.** Procédé de l'une quelconque des revendications précédentes, dans lequel l'artère sanguine est une artère radiale (320) au niveau d'un poignet (310).

**10.** Système pour effectuer une oxymétrie de pouls, le système comprenant :

un dispositif portable (110) pouvant fonctionner pour enregistrer de manière simultanée un électrocardiogramme, ECG et un photopléthysmogramme, PPG, dans lequel le PPG est mesuré au niveau d'une artère sanguine ; et
au moins un processeur (220, 710) couplé en communication au dispositif portable (110) et pouvant fonctionner pour :

analyser l'ECG et le PPG pour déterminer un temps de transit du pouls, PTT, une fréquence du pouls, PR, et un paramètre de diamètre, le paramètre de diamètre comportant l'un d'un diamètre de l'artère sanguine et d'un changement du diamètre de l'artère sanguine ; et
déterminer, sur la base du PTT, de la PR et du paramètre de diamètre, une pression sanguine, BP, en utilisant un modèle prédéfini, dans lequel le modèle prédéfini établit une relation entre le PTT, la PR, le paramètre de diamètre et la BP.

**11.** Système de la revendication 10, dans lequel l'au moins un processeur (220, 710) peut fonctionner pour effectuer le procédé de l'une quelconque des revendications 2 à 6.

**12.** Système de la revendication 10 ou 11, dans lequel le PPG comporte une intensité d'une lumière réfléchie par l'artère sanguine.

**13.** Système de la revendication 12, dans lequel une longueur d'onde de la lumière est isosbestique par rapport à une absorption de lumière par le sang dans l'artère sanguine.

**14.** Système de l'une quelconque des revendications 10 à 13, dans lequel le changement du diamètre de l'artère sanguine est déterminé en utilisant l'équation

$$\left(\frac{AC}{DC}\right) = c * \Delta\,d$$

, dans laquelle DC est une composante de courant continu du PPG, AC est une composante de courant alternatif du PPG, c est un coefficient d'absorption du sang dans l'artère sanguine, et $\Delta$ d est le changement du diamètre de l'artère sanguine.

**15.** Support de stockage non transitoire lisible par ordinateur sur lequel sont incorporées des instructions, qui, lorsqu'elles sont exécutées par au moins un processeur (220, 710), effectuent les étapes d'un procédé selon l'une quelconque des revendications 1 à 9.

EP 3 493 734 B1

Mobile Device
140

Computing Cloud
150

Patient
130

100

120

110

**FIG. 1**

**FIG. 2**

EP 3 493 734 B1

**FIG. 3**

EP 3 493 734 B1

**FIG. 4**

EP 3 493 734 B1

PPG

Blood vessel diameter

**FIG. 5**

EP 3 493 734 B1

600

Record, by a wearable device, substantially simultaneously an
electrocardiogram (ECG) and photoplethysmogram (PPG),
wherein the PPG is measured at a blood artery
602

Analyze, by at least one processor, the ECG and the PPG to
determine a pulse transit time (PTT), a pulse rate (PR), and a
diameter parameter, wherein the diameter parameter includes
one of a diameter of the blood artery and a change in the
diameter of the blood artery
604

Determine, by the at least one processor and using a pre-defined
model, a blood pressure (BP) based on the PTT, the PR, and the
diameter of parameter, wherein the pre-defined model establishes
a relationship between the PTT, the PR, the diameter parameter,
and the BP
606

# FIG. 6

700

790

| Processor Units
710 | | Output
Devices
750 |

| Main Memory
720 | | User Input
Devices
760 |

| Mass Data
Storage
730 | | Graphics Display
System
770 |

| Portable
Storage Device
740 | | Peripheral Devices
780 |

# FIG. 7

**EP 3 493 734 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 73866615
- US 73863615
- US 73871115
- US 2009163821 A1
- US 2015366469 A1
- US 8602997 B2
- US 6527725 B1
- US 2017202459 A1